# EUROPÄISCHE PATENTSCHRIFT

(11) **EP 0 543 123 B1**
(45) Veröffentlichungstag und Bekanntmachung des Hinweises auf die Patenterteilung: **20.03.1996**
(21) Anmeldenummer: 92116831.6
(22) Anmeldetag: 01.10.1992
(51) Int. Cl.: A61B 17/39

(54) **Medizinisches Hochfrequenz-Koagulationsinstrument**
Medical high frequency coagulation device
Instrument médical de coagulation à haute fréquence

(30) Priorität: 19.11.1991 DE 4138115
(43) Veröffentlichungstag der Anmeldung: 26.05.1993
(73) Patentinhaber: DELMA ELEKTRO-UND MEDIZINISCHE APPARATEBAU GESELLSCHAFT mbH, D-78505 Tuttlingen (DE)
(72) Erfinder: Hagen, Alfred, W-7200 Tuttlingen/Nendingen (DE)
(74) Vertreter: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) Entgegenhaltungen:
- EP-A- 0 067 680
- EP-A- 0 455 321
- EP-A- 0 521 501
- US-A- 4 040 426
- US-A- 4 326 529
- US-A- 4 682 596

## Beschreibung

Die Erfindung betrifft ein medizinisches Hochfrequenz-Koagulationsinstrument, wie es aus dem Dokument EP-A-0 521 501 (Stand der Technik gemäß Artikel 54(3) EPÜ) bekannt ist.

Bei diesem bekannten elektro-chirurgischen Behandlungsinstrument arbeiten die beiden Koagulationselektroden mit einer konzentrisch zu ihnen angeordneten Schneidelektrode zusammen. Die Koagulationselektroden werden im Gebrauch auf das zu koagulierende Gewebe bzw. Gefäß aufgelegt, worauf dann mittels eines Steuergerätes der Hochfrequenz-Koagulationsstrom solange eingeschaltet wird, bis durch den Stromfluß zwischen den beiden Koagulationselektroden die gewünschte Koagulation herbeigeführt worden ist.

Das Ziel der Erfindung besteht darin, ein weiteres Hochfrequenz-Koagulationsinstrument zu schaffen. Insbesondere soll dieses Instrument eine Beseitigung von im Operationsbereich freiwerdenden Gewebeteilen und/oder Flüssigkeiten ermöglichen.

Zur Lösung dieser Aufgabe sind die Merkmale des Patentanspruchs 1 vorgesehen. Vorteilhafte Weiterbildungen der Erfindung sind in den Ansprüchen 2 bis 8 definiert.

Die Erfindung nutzt also den Zwischenraum zwischen den beiden rohrförmigen Zuleitungen und dem Innenraum des inneren Stromzuführungsrohrs aus, um gleichzeitig mit dem Koagulationsvorgang aber auch davor oder danach eine Spülflüssigkeit zum Operationsfeld leiten und von dort Flüssigkeit absaugen zu können. Anders herum ausgedrückt, werden die Wände der Flüssigkeitskanäle dazu ausgenutzt, zusätzlich einen Koagulationsstrom zum distalen Ende des Instrumentenschafts zu führen.

Im Anschlußteil werden die elektrischen Ronrzuführleitungen in geeigneter Weise mit elektrischen Kontakten verbunden, denen über ein geeignetes Hochfrequenzgerst der Koagulationsstrom zugeführt werden kann. Weiter werden die Flüssigkeits-Zufuhr- bzw. -Abfuhrkanäle innerhalb des Anschlußteils in geeigneter Weise mit Flüssigkeits-Zufuhr- bzw. -Abfuhrleitungen verbunden. Eine besonders vorteilhafte Ausführungsform für einen derartigen Anschluß ist durch Anspruch 9 gekennzeichnet.

Insbesondere aus Gründen der Reinigung aber auch der Reparatur oder des Ersatzes ist eine lösbare Anordnung des Instrumentenschaftes am Anschlußteil gemäß Anspruch 10 bevorzugt.

Um die Flüssigkeitszu- und/oder -abfuhr auf einfachste Weise in Gang setzen und auch wieder unterbrechen zu können, sind die Ausführungsformen nach Anspruch 11 oder 12 vorteilhaft. Besonders bevorzugt ist es, wenn nach Anspruch 13 die Koagulations-Senkelektroden mit den zugeordneten Rohrzuführungsleitungen einstückig sind.

Die Erfindung wird im folgenden beispielsweise anhand der Zeichnung beschrieben; in dieser zeigt:
- Fig. 1: einen Axialschnitt durch das Instrumentenschaft eines erfindungsgemäßen Koagulationsinstrumentes im distalen Bereich,
- Fig. 2: einen Schnitt nach Linie II-II in Fig. 1,
- Fig. 3: einen entsprechenden Schnitt durch das erfindungsgemäße Instrument, wobei ein gegenüber den Fig.1 und 2 verkleinerter Maßstab gewählt ist, und
- Fig. 4: eine teilweise geschnittene Seitenansicht des Gegenstandes der Fig. 3 .

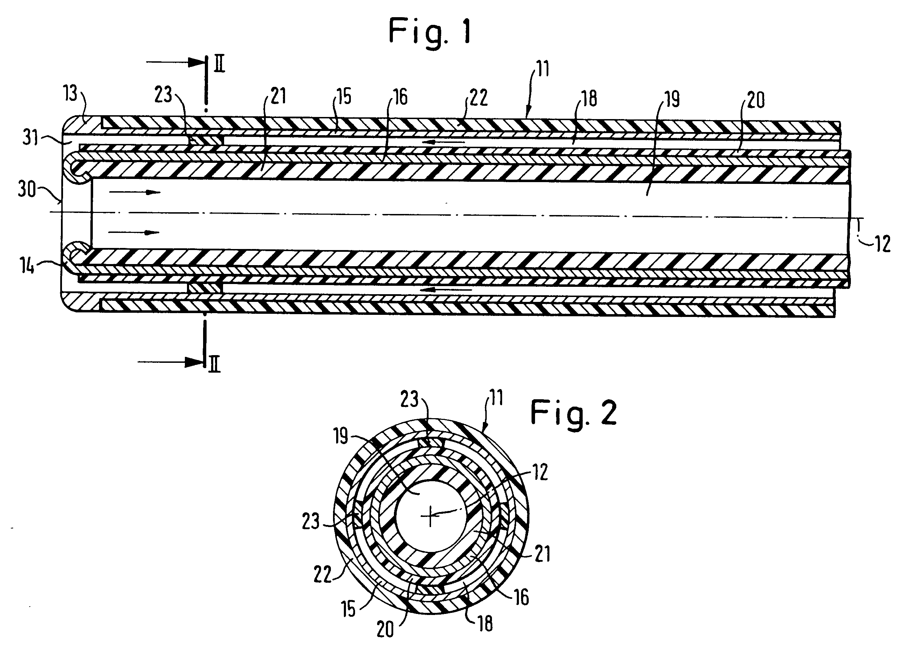

Nach Fig. 1 sind am vorderen Ende eines eine Achse 12 aufweisenden kreiszylindrischen Instrumentenschafts 11 zwei ringförmige Koagulationselektroden 13, 14 konzentrisch zueinander angeordnet. Die Elektroden sind in der dargestellten Weise abgerundet und bei diesem Ausführungsbeispiel am distalen Ende 30 bündig miteinander.

Nach hinten gehen die Elektroden 13, 14 bevorzugt einstückig in mit ihnen und der Schaftachse 12 konzentrische Rohrzuführungsleitungen 15, 16 über, welche mit isolierenden Schrumpfschläuchen 22 bzw. 20 überzogen sind. Die innere Rohrzuführungsleitung 16 ist darüber hinaus auf ihrer Innenseite mit einem Isolierschlauch 21 abgedeckt. Wichtig ist, daß die Isolierungen 20, 21, 22 sich nur bis zu den Ringelektroden 13, 14, nicht aber auch über diese erstrecken. Die metallischen Oberflächen der Ringelektroden 13, 14 müssen nach vorne und ggf. auch etwas zur Seite freiliegen, damit sie ihre Wirksamkeit entfalten können.

Zwischen den radial gegenüberliegenden Wänden der Rohrzuführungsleitungen 15, 16 bzw. des die innere Rohrzuführungsleitung 16 überziehenden Schrumpfschlauches 20 befindet sich ein ringzylindrischer Flüssigkeitszufuhrkanal 18, durch den hindurch in Pfeilrichtung eine geeignete Spülflüssigkeit zu der ringförmigen Austrittsöffnung 31 am distalen Ende 30 geleitet werden kann.

Zwischen der inneren Wand der Rohrzuführungsleitung 15 und der Außenwand des Schrumpfschlauches 20 können vorzugsweise isolierende Abstandshalter 23 angeordnet sein, welche einen definierten Abstand der beiden Rohranordnungen gewährleisten und mechanische Schwingungen zwischen ihnen vermeiden. Über den Umfang sind nach Fig. 2 verteilt z.B. vier derartige Abstandshalter 23 angeordnet, welche in Umfangsrichtung eine so geringe Ausdehnung haben, daß der axiale Flüssigkeitsdurchgang dazwischen möglichst wenig behindert wird. Derartige Abstandshalteranordnungen können an mehreren Stellen über die Länge des Instrumentenschaftes 11 verteilt vorgesehen sein.

Der Durchmesser der inneren Rohrzuführungsleitung 16 bzw. des innen darauf aufgebrachten Kunststoffschlauches 21 ist so groß gewählt, daß ein zentraler Flüssigkeits-Abfuhrkanal 19 vorliegt, durch den aus dem Operationsbereich in Richtung der Pfeile Flüssigkeit abgesaugt werden kann.

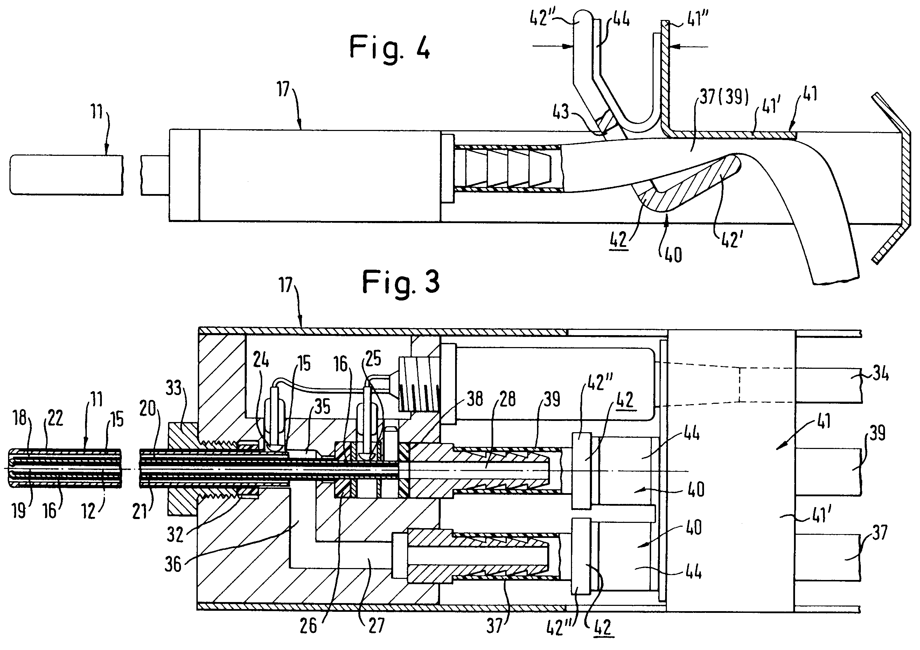

Nach Fig. 3 ist das hintere Ende des Instrumentenschafts 11 so ausgebildet, daß die innere Rohrzuführungsleitung 16 deutlich über die äußere Rohrzuführungsleitung 15 axial nach hinten vorsteht. Das so ausgebildete Ende des Instrumentenschafts 11 ist in eine dazu komplementäre Bohrung 32 eines Anschlußblockes 17 eingesteckt und dort beispielsweise mittels einer geeignet ausgebildeten Klemmschraube 33 festgelegt.

Die Isolierschichten 22 bzw. 20 sind im hinteren Bereich entfernt, so daß dort im Anschlußblock 17 vorgesehene radiale Kontakte 24, 25 mit den Rohrzuführungsleitungen 15, 16 in elektrische Verbindung treten können. Den Kontakten 24, 25 kann über ein an ein nicht dargestelltes Hochfrequenzgerät angeschlossenes Kabel 34 der für die Koagulation erforderliche Hochfrequenzstrom zugeführt werden.

Rund um das über die Rohrzuführungsleitung 15 nach hinten vorstehende Ende der Rohrzuführungsleitung 16 ist ein ringförmiger Flüssigkeits-Zufuhrraum 35 vorgesehen, der über einen Radialkanal 36 an eine Flüssigkeits-Zufuhrleitung 27 angeschlossen ist, der von außen z.B. über einen Schlauch 37 eine geeignete Spülflüssigkeit zugeführt wird.

An seinem hinteren Ende ist der Ringraum 35 durch eine Ringdichtung 26 gegenüber dem Bereich, wo der Kontakt 25 an die Rohrzuführungsleitung 16 angelegt ist, abgedichtet.

Das hintere Ende der Rohrzuführungsleitung 16 liegt axial an einer Ringdichtung 38 an und ist mit einer Flüssigkeits-Abfuhrleitung 28 axial ausgerichtet, die in der dargestellten Weise mit einem Flüssigkeits-Abfuhrschlauch 39 verbunden ist.

Im hinteren Bereich des Anschlußblockes 17, wo die Schläuche 37, 39 zu den Leitungen 27, 28 verlaufen, sind zwei sehr einfach herzustellende und arbeitende Ventile 40 gemäß Fig. 4 eingebaut, mit denen die Flüssigkeitszu- und -abfuhr individuell geregelt werden kann. Jedes Ventil 40 weist einen feststehenden winkelförmigen Anschlag 41 und einen verschwenkbaren Winkel-Klemmhebel 42 auf. Der Winkel-Klemmhebel 42 besitzt einen Klemmschenkel 42' und einen abgewinkelten Betätigungsschenkel 42'' auf, in dem eine Durchführungsbohrung 43 für den zugeordneten Schlauch 37 bzw. 39 vorgesehen ist. Darüber erstreckt sich zwischen dem Betätigungsschenkel 42'' und einem nach oben abgewinkelten Teil 41'' des Anschlages 41 eine V-förmige Spreizfeder 44, die mit dem Betätigungsschenkel 42'' und dem Teil 41'' verschweißt ist. In der entspannten Lage gemäß Fig. 3 und 4 schwenkt die Spreizfeder 44 den Klemmschenkel 42' nach oben, so daß der Schlauch 37 bzw. 39 gegen den parallel zu ihm liegenden Schenkel 41' des Anschlages 41 gedrückt und in der dargestellten Weise abgeklemmt wird, so daß der Flüssigkeitsdurchgang unterbunden ist.

Wenn von Hand zwischen dem oberen Anschlagschenkel 41'' und dem Betätigungsschenkel 42'' mit den Fingern eine Kraft in Richtung der beiden in Fig. 4 eingezeichneten Pfeile ausgeübt wird, wird die Spreizfeder 44 zusammengedrückt und dadurch die Abklemmung des Schlauches 37 bzw. 39 aufgehoben, indem der Klemmschenkel 42' nach unten geschwenkt wird.

Auf diese Weise kann der durch den Schlauch 37 bzw. 39 zugeführte Spülflüssigkeits- bzw. abgeführte Flüssigkeitsstrom auf einfachste Weise unterbrochen oder zum Instrumentenschaft 11 geführt werden. Scharniere oder sonstige aufwendige Ventilanordnungen sind hierfür nicht erforderlich.

## Patentansprüche

1. Medizinisches Hochfrequenz-Koagulationsinstrument mit einem vorzugsweise kreiszylindrischen Instrumentenschaft (11), welcher am distalen Ende zwei ineinander und vorzugsweise konzentrisch zueinander und zur Schaftachse (12) angeordnete, elektrisch leitende Koagulations-Ringelektroden (13, 14) aufweist, von denen nach hinten ineinander und vorzugsweise konzentrisch zueinander und zur Schaftachse (12) angeordnete, elektrisch leitende, gegeneinander isolierte Rohrzuführungsleitungen (15, 16) ausgehen, deren proximales Ende mit einem Anschlußstück (17) zur Zuführung des erforderlichen Hochfrequenzstromes verbunden ist, und wobei
zwischen den Wänden der beiden Rohrzuführungsleitungen (15, 16) und im Inneren der inneren Rohrzuführungsleitung (16) axiale Flüssigkeits-Zufuhr- bzw. -Abfuhrkanäle (18, 19) vorgesehen sind.

2. Instrument nach Anspruch 1, dadurch gekennzeichnet, daß zwischen den radial gegenüberliegenden Wänden der Rohrzuführungsleitungen (15, 16) außer den Flüssigkeits-Zufuhr- bzw. -Abfuhrkanälen (18, 19) eine elektrische Isolation vorgesehen ist.

3. Instrument nach Anspruch 2, dadurch gekennzeichnet, daß als elektrische Isolation auf der Außenseite der inneren Rohrzuführungsleitung (16) eine erste Isolierschicht (20), vorzugsweise in Form eines Schrumpfschlauches, vorgesehen ist.

4. Instrument nach einem der vorhergehenden Ansprüche, dadurch gekennzeichnet, daß zwischen der inneren Wand der inneren Rohrzuführungsleitung (16) und dem inneren Flüssigkeits-Zufuhr- bzw. Abfuhrkanal (19) eine Isolation vorgesehen ist.

5. Instrument nach Anspruch 4, dadurch gekennzeichnet, daß als Isolation auf der Innenseite der inneren Rohrzuführungsleitung (16) eine zweite Isolierschicht (21), vorzugsweise in Form eines Kunststoffrohres, vorgesehen ist.

6. Instrument nach einem der vorhergehenden Ansprüche, dadurch gekennzeichnet, daß auf der Außenseite der äußeren Rohrzuführungsleitung (15) eine dritte Isolierschicht (22), vorzugsweise in Form eines Schrumpfschlauches, vorgesehen ist.

7. Instrument nach einem der Ansprüche 1 bis 6, dadurch gekennzeichnet, daß zwischen den radial gegenüberliegenden Wänden der Rohrzuführungsleitungen (15, 16) bzw. der ersten Isolierschicht (20) radiale Abstandshalter (23) in solcher Ausbildung und/oder Anordnung vorgesehen sind, daß sie die axiale Flüssigkeitsströmung nicht verhindern.

8. Instrument nach einem der Ansprüche 3 oder 5 bis 7, dadurch gekennzeichnet, daß die Isolierschichten (20, 21, 22) im Bereich der Koagulationselektroden (13, 14) enden, so daß letztere nach vorn und ggf. auch seitlich freiliegen.

9. Instrument nach einem der vorhergehenden Ansprüche, dadurch gekennzeichnet, daß die innere Rohrzuführungsleitung (16) im Anschlußstück (17) über die äußere Rohrzuführungsleitung (15) nach hinten vorsteht und daß an die abisolierten Rohrzuführungsleitungen (15, 16) radial von außen elektrische Kontakte (24, 25) angelegt sind, die an eine Hochfrequenzquelle anschließbar sind, wobei die Flüssigkeits-Zufuhr- bzw. -Abfuhrkanäle (18, 19) an axial unterschiedlichen Stellen, die gegeneinander abgedichtet (26) sind, an Flüssigkeits-Zufuhr- bzw. -Abfuhrleitungen (27, 28) angeschlossen sind.

10. Instrument nach einem der vorhergehenden Ansprüche, dadurch gekennzeichnet, daß der Instrumentenschaft (11) mit dem Anschlußstück (17) lösbar verbunden ist.

11. Instrument nach einem der vorhergehenden Ansprüche, dadurch gekennzeichnet, daß im Anschlußstück (17) wenigstens ein Ventil und vorzugsweise zwei Ventile (40) zur Steuerung der Flüssigkeitszu- und/oder -abfuhr vorgesehen sind.

12. Instrument nach Anspruch 11, dadurch gekennzeichnet, daß das Ventil (40) einen festen Anschlag (41) und einem Winkel-Klemmhebel (42) aufweist, der einen Klemmschenkel (42') und einen mit einer schlauchdurchgangsbohrung (43) versehenen Betätigungsschenkel (42'') aufweist, welcher über eine Spreizfeder (44) mit einem Schenkel (41'') des Anschlages (41) fest verbunden ist, die den Klemmschenkel (42'') derart gegen einen weiteren Schenkel (41') des Anschlages (41) drückt, daß der dazwischen hindurchgeführte Schlauch (37 bzw. 39) abgeklemmt wird.

13. Instrument nach einem der vorhergehenden Ansprüche, dadurch **gekennzeichnet,** daß die Koagulations-Ringelektroden (13, 14) mit den zugeordneten Rohrzuführungsleitungen (15, 16) einstückig sind.

## Claims

1. Medical high frequency coagulation instrument with a preferably circular cylindrical instrument shaft (11) which has, at the distal end, two electrically conductive coagulation ring electrodes (13, 14) which are arranged inside one another and preferably concentric to one another and to the shaft axis (12), and from which electrically conductive, mutually insulated tubular feedlines (15, 16) extend rearwardly and are preferably arranged concentric to one another and to the shaft axis (12), with their proximal end being connected to a connection piece (17) for the supply of the required high frequency current, and wherein axial liquid supply and removal channels (18, 19) are respectively provided between the walls of the two tubular feedlines (15, 16) and in the interior of the inner tubular feedline (16).

2. Instrument in accordance with claim 1, characterized in that an electrical insulation is provided between the radially oppositely disposed walls of the tubular feedlines (15, 16) in addition to the liquid supply and removal channels (18, 19).

3. Instrument in accordance with claim 2, characterized in that a first insulating layer (20) preferably in the form of a shrinkable hose is provided on the outer side of the inner tubular feedline (16) as electrical insulation.

4. Instrument in accordance with one of the preceding claims, characterized in that an insulation is provided between the inner wall of the inner tubular feedline (16) and the inner liquid supply or removal channel (19).

5. Instrument in accordance with claim 4, characterized in that a second insulating layer (21) preferably in the form of a plastic tube is provided on the inner side of the inner tubular feedline (16) as insulation.

6. Instrument in accordance with one of the preceding claims, characterized in that a third insulating layer (22), preferably in the form of a shrinkable hose, is provided on the outer side of the outer tubular feedline (15).

7. Instrument in accordance with one of the claims 1 to 6, characterized in that radial spacers (23) are provided between the radially oppositely disposed walls of the tubular feedlines (15, 16) and/or of the first insulating layer (20) in such a design and/or arrangement that they do not prevent the axial flow of liquid.

8. Instrument in accordance with one of the claims 3 or 5 to 7, characterized in that the insulating layers (20, 21, 22) terminate in the region of the coagulation electrodes (13, 14) so that the latter are exposed to the front and optionally also to the side .

9. Instrument in accordance with one of the preceding claims, characterized in that the inner tubular feedline (16) in the connection piece (17) projects rearwardly in the connection piece(17) beyond the outer tubular feedline (15), and in that electrical contacts are applied radially from the outside to the de-insulated tubular feedlines (15, 16) and are connectable to the high frequency source, with the liquid supply and removal channels (18, 19) being connected at axially differing positions, which are sealed relative to one another (26), to the liquid supply and discharge lines (27, 28).

10. Instrument in accordance with one of the preceding claims, characterized in that the instrument shaft (11) is releasably connected to the connection piece (17).

11. Instrument in accordance with one of the preceding claims, characterized in that at least one valve and preferably two valves (40) are provided in the connection piece (17) for the control of the liquid supply and/or removal.

12. Instrument in accordance with claim 11, characterized in that the valve (40) has a fixed abutment (41) and a cranked clamping lever (42) which has a clamping limb (42') and an actuating limb (42'') provided with an aperture (43) for the passage of a hose, the actuating limb being connected via a spreading spring (44) with one limb (41'') of the abutment (41) which presses the clamping limb (42'') against a further limb (41') of the abutment (41) in such a way that the hose (37, 39 respectively) which passes through it, is clamped off.

13. Instrument in accordance with one of the preceding claims, characterised in that the coagulation ring electrodes (13, 14) are formed in one piece with the associated tubular feedlines (15, 16).

## Revendications

1. Instrument médical de coagulation à haute fréquence comportant un tronc d'instrument (11) de préférence cylindrique circulaire qui présente à l'extrémité distale deux électrodes annulaires de coagulation (13, 14) électriquement conductrices et agencées l'une dans l'autre et de préférence concentriquement l'une par rapport à l'autre et par rapport à l'axe de tronc (12), depuis lesquelles partent vers l'arrière des conduites d'amenée tubulaires (15, 16) électriquement conductrices et isolées l'une par rapport à l'autre et agencées l'une derrière l'autre et de préférence concentriquement l'une par rapport à l'autre et par rapport à l'axe de tronc (12), dont l'extrémité proximale est reliée à un élément de raccordement (17) pour l'amenée du courant à haute fréquence nécessaire, et dans lequel il est prévu entre les parois des deux conduites d'amenée tubulaires (15, 16) et dans l'intérieur de la conduite d'amenée tubulaire intérieure (16) des canaux d'amenée ou d'évacuation de liquide axiaux (18, 19).

2. Instrument selon la revendication 1, caractérisé en ce que, outre les canaux d'amenée et d'évacuation de liquide (18, 19), il est prévu une isolation électrique entre les parois radialement en vis-à-vis des conduites d'amenée tubulaires (15, 16).

3. Instrument selon la revendication 2, caractérisé en ce qu'il est prévu, en tant qu'isolation électrique sur la face extérieure de la conduite d'amenée tubulaire intérieure (16) une première couche d'isolation (20), de préférence sous forme d'un tuyau rétractable.

4. Instrument selon l'une quelconque des revendications précédentes, caractérisé en ce qu'il est prévu une isolation entre la paroi intérieure de la conduite d'amenée tubulaire intérieure (16) et le canal d'amenée ou d'évacuation de liquide intérieur (19).

5. Instrument selon la revendication 4, caractérisé en ce qu'il est prévu en tant qu'isolation sur la face intérieure de la conduite d'amenée tubulaire intérieure (16) une seconde couche d'isolation (21), de préférence sous forme d'un tube en matière plastique.

6. Instrument selon l'une quelconque des revendications précédentes, caractérisé en ce qu'il est prévu sur la face extérieure de la conduite d'amenée tubulaire extérieure (15) une troisième couche d'isolation (22), de préférence sous forme d'un tuyau rétractable.

7. Instrument selon l'une quelconque des revendications 1 à 6, caractérisé en ce qu'il est prévu entre les parois, radialement en vis-à-vis, des conduites d'amenée tubulaires (15, 16) ou de la première couche d'isolation (20) des éléments d'écartement radiaux (23), avec une réalisation et/ou un agencement de telle sorte qu'ils n'empêchent pas le courant de liquide axial.

8. Instrument selon l'une quelconque des revendications 3 ou 5 à 7, caractérisé en ce que les couches d'isolation (20, 21, 22) se terminent dans la région des électrodes de coagulation (13, 14), de sorte que ces dernières sont libres vers l'avant, et le cas échéant également vers le côté.

9. Instrument selon l'une quelconque des revendications précédentes, caractérisé en ce que la conduite d'amenée tubulaire intérieure (16) dépasse dans la partie de raccordement (17) vers l'arrière au-delà de la conduite d'amenée tubulaire extérieure (15), et en ce que sur les conduites d'amenée tubulaires dénudées (15, 16) sont appliqués radialement depuis l'extérieur des contacts électriques (24, 25) qui peuvent être branchés à une source à haute fréquence, les canaux d'amenée et d'évacuation de liquide (18, 19) étant raccordés, à des emplacements axialement différents qui sont étanchés les uns par rapport aux autres (26), aux conduites d'amenée ou d'évacuation de liquide (27, 28).

10. Instrument selon l'une quelconque des revendications précédentes, caractérisé en ce que le tronc d'instrument (11) est relié de façon détachable à l'élément de raccordement (17).

11. Instrument selon l'une quelconque des revendications précédentes, caractérisé en ce qu'il est prévu dans l'élément de raccordement (17) au moins une soupape ou de préférence deux soupapes (40) pour la commande de l'amenée et/ou de l'évacuation de liquide.

12. Instrument selon la revendication 11, caractérisé en ce que la soupape (40) présente une butée fixe (41) et un levier de serrage angulaire (42) qui présente un bras de serrage (42') et un bras d'actionnement (42'') pourvu d'un perçage de passage de tuyau (43), ce bras d'actionnement étant relié fermement à un bras (41'') de la butée (41) via un ressort d'écartement (44) qui presse le bras de serrage (42'') contre un autre bras (41') de la butée (41), de telle sorte que le tuyau (37 ou 39) mené entre les deux est pincé.

13. Instrument selon l'une quelconque des revendications précédentes, caractérisé en ce que les électrodes annulaires de coagulation (13, 14) sont réalisées en une seule pièce avec les conduites d'amenée tubulaires associées (15, 16).
